# EUROPEAN PATENT APPLICATION

(11) **EP 4 524 145 A1**
(43) Date of publication of application: **19.03.2025**
(21) Application number: 23820125.5
(22) Date of filing: 09.06.2023
(51) Int. Cl.: C07H 19/12, A61K 31/706, C07F 7/18

(54) **METHOD FOR PREPARING 4?-THIO-5-AZA-2?-DEOXYCYTIDINE**

(30) Priority: 10.06.2022 KR 20220071014
(71) Applicant: Pinotbio, Inc., Suwon-si, Gyeonggi-do 16506 (KR); ST Pharm Co., Ltd., Siheung-si, Gyeonggi-do 15086 (KR)
(72) Inventor: JUNG, Doo Young, Suwon-si, Gyeonggi-do 16506 (KR); LEE, Jin Soo, Suwon-si, Gyeonggi-do 16506 (KR); CHO, Hyun Yong, Suwon-si, Gyeonggi-do 16506 (KR); KIM, Ah Reum, Ansan-si, Gyeonggi-do 15610 (KR); SEO, Mun Sik, Ansan-si, Gyeonggi-do 15610 (KR); CHOI, Jun Young, Ansan-si, Gyeonggi-do 15610 (KR)
(74) Representative: Prüfer & Partner mbB Patentanwälte · Rechtsanwälte
(86) International application number: PCT/KR2023/007909
(87) International publication number: WO 2023/239190

(57) **Abstract**

The present invention relates to a method for preparing 4'-thio-5-aza-2'-deoxycytidine which exhibits DNMT1 inhibitory activity. The production method according to the present invention can enhance reaction efficiency through efficient process development and can stereoselectively prepare 4'-thio-5-aza-2'-deoxycytidine, and thus is economical and appropriate for mass production.

## Description

### [Technical Field]

The present invention relates to a method for preparing 4'-thio-5-aza-2'-deoxycytidine which exhibits DNMT1 inhibitory activity. Specifically, the present invention relates to a method for stereoselectively preparing 4'-thio-5-aza-2'-deoxycytidine.

### [Background Art]

4'-Thio-5-aza-2'-deoxycytidine (hereinafter, referred to as "5-aza-T-dCyd") is a novel DNMT1 (DNA (cytosine-5)-methyltransferase 1) inhibitor that has been initially clinical evaluated by the National Cancer Institute (NCI), and is currently clinical as a candidate for treatment of blood cancer or solid cancer.

Meanwhile, 5-aza-T-dCyd is a β-anomer as represented by the following Chemical Formula I, and in order to use 5-Aza-T-dCyd as a pharmaceutical raw material, a method for stereoselectively preparing the 5-aza-T-dCyd is most important.

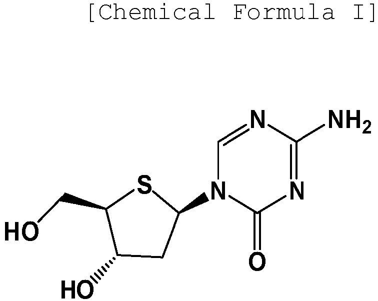

International Publication No. WO2019/152459 discloses a method for preparing 5-aza-T-dCyd. Specifically, International Publication No. WO2019/152459 (FIGS. 4 and 5) shows a conventional preparation method, and as represented in the following reaction scheme, the preparation method has a problem in that it is not suitable for mass production because a purification process is essential to obtain the β-anomer.

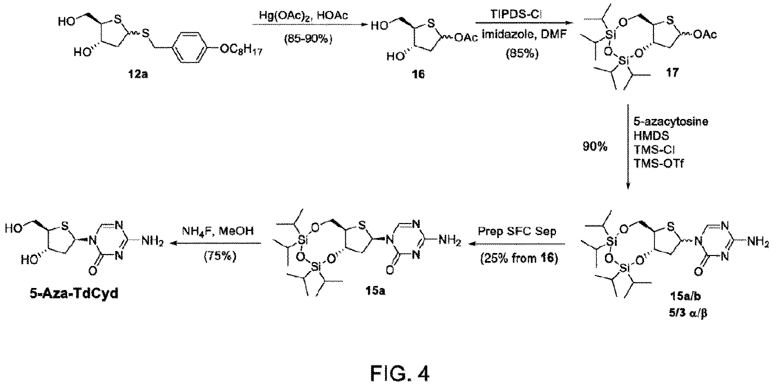

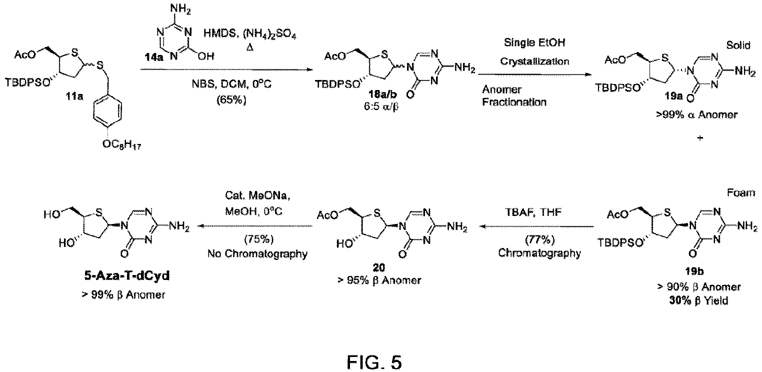

Meanwhile, International Publication No. WO2019/152459 discloses a new preparation method for improving the above problem (FIG. 7 below).

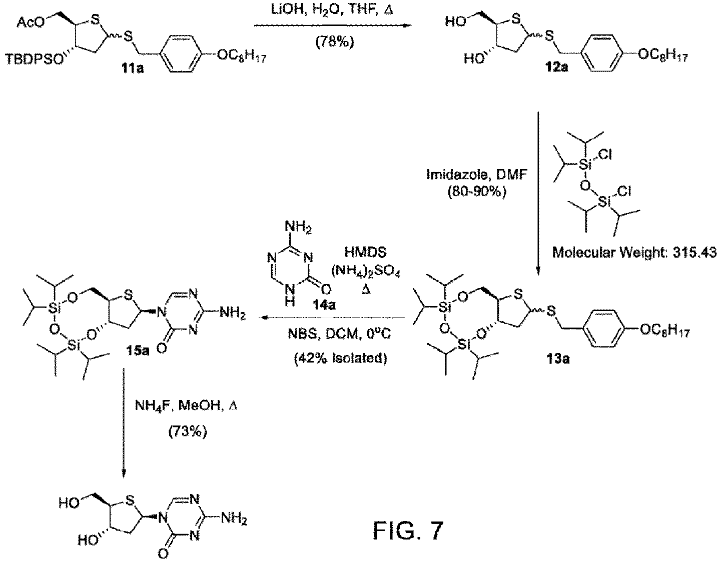

According to the preparation method of the above reaction scheme, the β-anomer may be predominantly prepared, but a ratio of the β-anomer and the α-anomer is 6:1, so that an additional process is still required to obtain 100% of the β-anomer. In other words, all of the preparation methods disclosed in International Publication No. WO2019/152459 are not suitable for mass production, so that process improvement thereof is still required.

Therefore, there is an urgent need to improve the above inefficient preparation method and develop a novel preparation method capable of stereoselectively preparing the β-anomer of 5-aza-T-dCyd represented by Chemical Formula I.

### [Disclosure]

### [Technical Problem]

An object of the present invention is to provide a method for stereoselectively preparing 4'-thio-5-aza-2'-deoxycytidine, which is a β-anomer, capable of achieving a low production cost and an efficient process step suitable for mass production.

### [Technical Solution]

The present invention provides a method for stereoselectively preparing 4'-thio-5-aza-2'-deoxycytidine. Unless otherwise defined, all terms used herein have the same meaning as commonly understood by those skilled in the art to which the present invention pertains. All patent and non-patent documents mentioned throughout the disclosure of the present application are incorporated by reference in their entirety.

Specifically, the preparation method of the present invention includes the following steps (S-1) to (S-3):
(S-1) preparing a compound represented by the following Chemical Formula 2 from a compound represented by the following Chemical Formula 1;
(S-2) preparing a compound represented by the following Chemical Formula 3 from the compound represented by Chemical Formula 2; and
(S-3) preparing a compound represented by the following Chemical Formula I from the compound represented by Chemical Formula 3;
a method for preparing 4'-thio-5-aza-2'-deoxycytidine comprising the above: 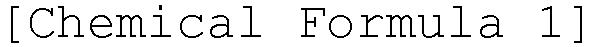 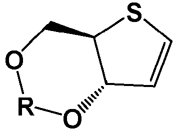 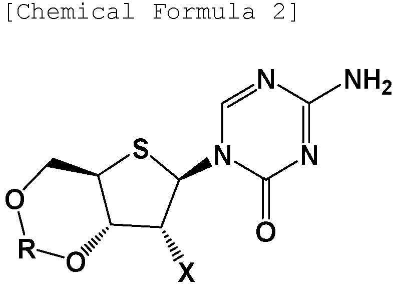 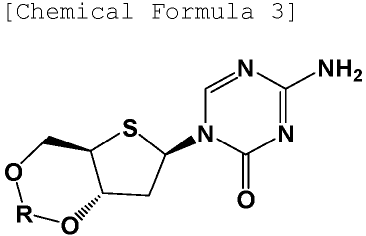 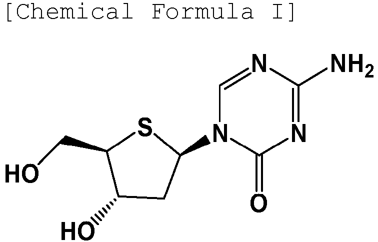 wherein
   R is a protecting group of diol including one or more Si atom, and
   X is halo.
   Hereinafter, each of steps (S-1) to (S-3) will be described separately.

### Step (S-1)

In the present invention, the step (S-1) is a step of preparing a compound represented by Chemical Formula 2 by introducing 5-azacytosine at a 1'-position and introducing halo at a 2'-position to the compound represented by Chemical Formula 1 having a double bond as shown in the following Reaction Scheme 1:

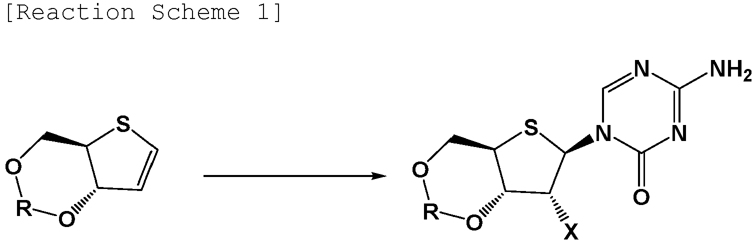

wherein
R is a protecting group of diol including one or more Si atom, and
X is halo.

In the present invention, since 5-azacytosine is β-selectively introduced at the 1'-position, 4'-thio-5-aza-2'-deoxycytidine, which is a target material, may be stereoselectively prepared through steps (S-2) and (S-3), which are the following processes. Therefore, unlike the conventional preparation method, the preparation method of the present invention does not require a separation process for obtaining the β-anomer, and thus is suitable for mass production.

According to an embodiment of the present invention, the compound represented by Chemical Formula 1 may be a compound represented by the following Chemical Formula 1a or a compound represented by the following Chemical Formula 1b: 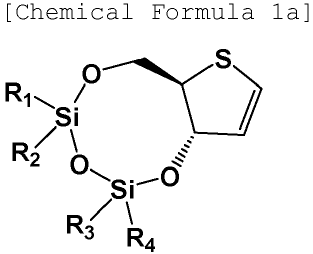 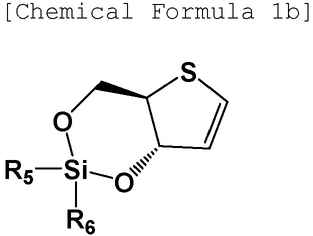 wherein
R₁ to R₆ are each independently -C₁-C₆ alkyl, 3-7 membered cycloalkyl, or aryl. For example, R₁ to R₆ may be methyl, ethyl, n-propyl, isopropyl, n-butyl, isobutyl, tertbutyl, cyclobutyl, cyclopentyl, cyclohexyl, or phenyl, but the present invention is not limited thereto.

In addition, according to the embodiment of the present invention, X may be -bromo (-Br) or -iodo(-I).

In the present invention, the reaction of introducing 5-azacytosine at the 1'-position is to react 5-azacytosine to the compound represented by Chemical Formula 1, which may be performed in the presence of hexamethyldisilane (HMDS). In addition, the reaction may be performed in the presence of ammonium sulfate. In addition, the reaction may be performed at 90 to 160°C, and more specifically at 110 to 130°C, but the present invention is not limited thereto. In addition, the reaction may be performed for 8 to 26 hours, and more specifically for 15 to 20 hours, but the present invention is not limited thereto.

In the present invention, the reaction of introducing halo to the 2'-position is to add N-iodosuccinimide (NIS) or N-bromosuccinimide (NBS), which may be performed in the presence of trimethylsilyl trifluoromethanesulfonate (TMSOTf), tert-butyldimethylsilyl trifluoromethanesulfonate (TBDMSOTf), trifluoromethanesulfonic acid (TfOH), or boron trifluoride diethyl etherate (BF₃·OEt₂). The reaction may be performed in an organic solvent, and for example, one or more solvents selected from the group consisting of acetonitrile, methylene chloride, ethylene chloride, ethyl acetate, toluene, chlorobenzene, chloroform, isopropyl acetate, tertbutyl acetate, and tetrahydrofuran may be used. In addition, the reaction may be performed at -10 to 40°C, and more specifically, at 0 to 25°C, but the present invention is not limited thereto. In addition, the reaction may be performed for 1 to 48 hours, and more specifically for 1 to 24 hours, but the present invention is not limited thereto.

According to the embodiment of the present invention, the compound represented by Chemical Formula 1 may be prepared from a compound represented by the following Chemical Formula 1c: 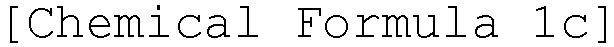 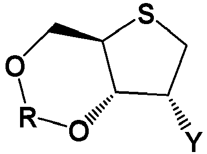 wherein
Y is methanesulfonate (-OMs), trifluoromethanesulfonate (-OTf), or toluenesulfonate (-OTs).

In addition, according to the embodiment of the present invention, the compound represented by Chemical Formula 1c may be prepared from a compound represented by the following Chemical Formula 1d:

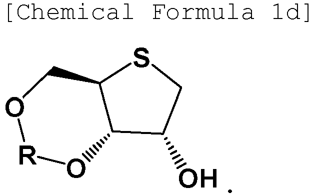

In addition, according to the embodiment of the present invention, the compound represented by Chemical Formula 1d may be prepared from a compound represented by the following Chemical Formula 1e:

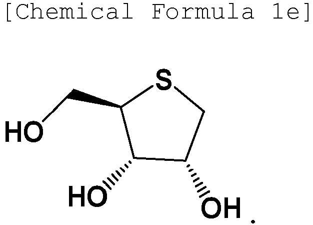

### Step (S-2)

In the present invention, the step (S-2) is a step of preparing a compound represented by Chemical Formula 3 by introducing hydrogen instead of halo at the 2'-position of the compound represented by Chemical Formula 2 as represented by the following Reaction Scheme 2: 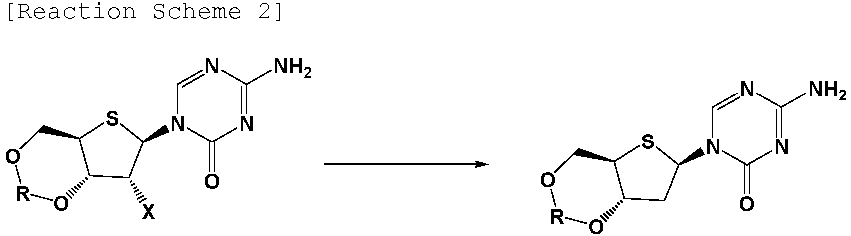 wherein R is as defined above.

According to the embodiment of the present invention, the step (S-2) may be performed by reacting the compound represented by Chemical Formula 2 with a radical hydrogen donor in the presence of a radical initiator.

The radical hydrogen donor may be tributyltin hydride (Bu₃SnH), triphenyltin hydride (Ph₃SnH), or triethylborane (Et₃B). However, the present invention is not limited thereto.

The radical initiator may be 2,2'-azobisisobutyronitrile (AIBN), 1,1'-azobis(cyclohexanecarbonitrile)isobutyronitrile (ABCN), 2,2'-azobis-2,4-dimethylvaleronitrile (ADMVN), or 2,2'-azobis(4-methoxy-2,4-dimethylvaleronitrile (V-70). However, the present invention is not limited thereto.

The reaction may use an organic solvent commonly used in the radical reaction. For example, as the solvent, one or more solvents selected from the group consisting of toluene, xylene, benzene, chlorobenzene, chloroform, 1,4-dioxane, and tetrahydrofuran may be used. Specifically, toluene may be used as the solvent, but the present invention is not limited thereto.

In addition, the reaction may be performed at 40 to 120°C, specifically at 45 to 115°C, and more specifically at 60 to 110°C, but the present invention is not limited thereto. In addition, the reaction may be performed for 1 to 48 hours, specifically for 1 to 24 hours, and more specifically for 1 to 4 hours, but the present invention is not limited thereto.

### Step (S-3)

In the present invention, the step (S-3) is a step of preparing a compound represented by Chemical Formula I through a deprotection reaction of the compound represented by Chemical Formula 3 as represented by the following Reaction Scheme 3: 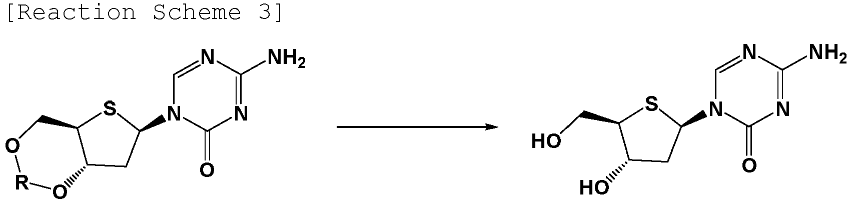 wherein R is as defined above.

According to the embodiment of the present invention, the reaction may be performed by adding ammonium fluoride (NH₄F), tetra-N-butylammonium fluoride (TBAF), hydrogen fluoride-pyridine (1:1), or triethylamine trihydrofluoride (TREAT-HF). However, the present invention is not limited thereto.

The reaction may use an organic solvent commonly used in the deprotection reaction. For example, as the solvent, one or more solvents selected from the group consisting of methanol, 1,4-dioxane, tetrahydrofuran, and acetonitrile may be used. Specifically, methanol may be used as the solvent, but the present invention is not limited thereto.

In addition, the reaction may be performed at 0 to 70°C, and more specifically at 25 to 65°C, but the present invention is not limited thereto. In addition, the reaction may be performed for 0.5 to 12 hours, and more specifically for 2 to 5 hours, but the present invention is not limited thereto.

In addition, according to the embodiment of the present invention, a process of crystallizing a product after the reaction may be further included. However, the present invention is not limited thereto.

### [Advantageous Effects]

The preparation method of the present invention may reduce production costs and is suitable for mass production through an efficient process step as compared to the conventional preparation method, by stereoselectively preparing 4'-thio-5-aza-2'-deoxycytidine, which is a β-anomer.

### [Best Mode]

Hereinafter, preferred examples are presented to help understand the present invention. However, the following examples are provided only to more easily understand the present invention, and the contents of the present invention are not limited by the examples.

### Preparation Example: Preparation of (6aR,9aS)-2,2,4,4-tetraisopropyl-6a,9a-dihydro-6H-thieno[3,2-f][1,3,5,2,4]trioxadisilocin

### Step 1: Preparation of (6aR,9R,9aS)-2,2,4,4-tetraisopropyltetrahydro-6H-thieno[3,2-f][1,3,5,2,4]trioxadisilocin-9-ol

(2R,3S,4R)-2-(Hydroxymethyl)tetrahydrothiophene-3,4-diol (1.58 g, 10.52 mmol) was dissolved in pyridine (21 mL), and then 1,3-dichloro-1,1,3,3-tetraisopropyldisiloxane (TIPDSCl₂, 11.33 mL, 36.3 mmol) was added thereto at 0°C, followed by stirring at room temperature for 12 hours. After completion of the reaction, ice was added to the reaction product to terminate the reaction, and ethyl acetate (20 mL) and water (20 mL) were added to the resultant, followed by extraction. An organic layer was extracted with a saturated aqueous sodium hydrogen carbonate solution (20 mL x 2) and a saturated aqueous sodium chloride solution (20 mL). The extracted solution was dried with sodium sulfate, filtered, and concentrated under reduced pressure. The residue obtained after concentration was dissolved by adding methylene chloride. Silica gel was added and then concentrated under reduced pressure once again. The residue adsorbed on the silica gel was subjected to silica gel column chromatography using a mixed solvent of ethyl acetate:hexane (1:15, v/v) as a developing solvent to obtain a target compound (2.07 g, 70%) in the form of a colorless syrup.

R_{f} = 0.25 (EtOAc/Hexane, 1:10).

¹H NMR (400 MHz, CDCl₃) *δ* 1.07-1.11 (28H, m, TIPDS), 2.65 (1H, s), 2.85 (1H, dd, *J* = 1.6, 12.1 Hz), 3.03 (1H, ddd, *J* = 1.0, 4.6, 12.1 Hz), 3.50 (1H, m), 3.91 (1H, dd, *J* = 4.5, 12.4 Hz), 4.03 (1H, dd, *J* = 3.3, 12.4 Hz), 4.23 (1H, dd, *J* = 3.9, 8.5 Hz), 4.34 (1H, m).

### Step 2: Preparation of (6aR,9R,9aS)-2,2,4,4-tetraisopropyltetrahydro-6H-thieno[3,2-f][1,3,5,2,4]trioxadisilocin-9-yl methanesulfonate

The compound (1.71 g, 4.35 mmol) obtained in Step 1 was dissolved in pyridine (22 mL). Methanesulfonyl chloride (0.44 mL, 5.66 mmol) was added at 0°C, and then stirred at room temperature for 5 hours. After completion of the reaction, ice was added to the resultant to terminate the reaction, and ethyl acetate (30 mL) and water (20 mL) were added to the resultant, followed by extraction. An organic layer was extracted with a saturated aqueous sodium hydrogen carbonate solution (30 mL x 3) and a saturated aqueous sodium chloride solution (30 mL). The extracted solution was dried with sodium sulfate, filtered, and concentrated under reduced pressure. The residue obtained after concentration was dissolved by adding methylene chloride. Silica gel was added and then concentrated under reduced pressure once again. The residue adsorbed on the silica gel was subjected to silica gel column chromatography using a mixed solvent of ethyl acetate:hexane (1:10, v/v) as a developing solvent to obtain a target compound (1.89 g, 92%) in the form of a white solid.

R_{f} = 0.1 (EtOAc/Hexane, 1:10).

¹H NMR (400 MHz, CDCl₃) *δ* 1.00-1.10 (28H, m, TIPDS), 3.01 (1H, t, *J* = 15.3 Hz), 3.11 (1H, s), 3.20 (1H, dd, *J* = 4.3, 13.0 Hz), 3.51 (1H, dt, *J* = 2.5, 10.0 Hz), 3.93 (1H, dd, *J* = 1.7, 12.7 Hz), 4.06 (1H, dd, *J* = 2.8, 12.7 Hz), 4.27 (1H, dd, *J* = 3.2, 10.0 Hz), 5.28 (1H, dd, *J* = 3.6 Hz).

### Step 3: Preparation of (6aR,9aS)-2,2,4,4-tetraisopropyl-6a,9a-dihydro-6H-thieno[3,2-f][1,3,5,2,4]trioxadisilocin

The compound (1.13 g, 2.4 mmol) obtained in Step 2 was dissolved in dimethylformamide (10 mL). 1,5-Diazabicyclo(4.3.0)non-5-ene (DBN, 2 mL, 16.8 mmol) was added to the resultant, heated to 150°C, and stirred under reflux for 30 minutes. After completion of the reaction, the reaction mixture was cooled to room temperature, ethyl acetate (20 mL) was added thereto, and the resulting mixture was washed with a saturated aqueous sodium hydrogen carbonate solution (20 mL x 3). An organic layer was dried with sodium sulfate, filtered, and concentrated under reduced pressure. The residue obtained after concentration was subjected to silica gel column chromatography using hexane (100%) and a mixed solvent of hexane:ethyl acetate (10:1, v/v) as a developing solvent to obtain a target compound (502 mg, 56%) in the form of a colorless syrup.

R_{f} = 0.2 (Hexane, 100%).

¹H NMR (400 MHz, CDCl₃) *δ* 1.04-1.10 (28H, m, TIPDS), 3.79 (1H, t, *J* = 11.2 Hz), 3.87 (1H, m), 4.09 (1H, dd, *J* = 3. 7, 11.0 Hz), 5.43 (1H, m), 5.57 (1H, dd, *J* = 2.6, 5.9 Hz), 6.22 (1H, dd, *J* = 1.5, 5.9 Hz).

### Example: Preparation of 4'-thio-5-aza-2'-deoxycytidine

### Step 1: Preparation of 4-amino-1-((aR,8R,9R,9aR)-9-iodo-2,2,4,4-tetraisopropyltetrahydro-6H-thieno[3,2-f][1,3,5,2,4]trioxadisilocin-8-yl)-1,3,5-triazin-2(1H)-one

5-Azacytosine (225 mg, 2.00 mmol) and ammonium sulfate (6 mg, 0.46 mmol) were added to hexamethyldisilane (HMDS, 8.5 mL), and a suspension thereof was refluxed for 16 hours to obtain a homogeneous solution. After completion of the reaction, the solvent was removed under reduced pressure, and methylene chloride (4 mL) was added to the residue, followed by stirring. Thereafter, trimethylsilyl trifluoromethanesulfonate (TMSOTf, 0.33 mL, 1.80 mmol) was added to the compound at 0°C, and then stirred until the residue was dissolved. The reaction mixture was maintained at 0°C, and the compound obtained in Preparation Example (450 mg, 1.20 mmol) was dissolved in acetonitrile (8.0 mL) and added thereto, and then N-iodosuccinimide (NIS) (405 mg, 1.80 mmol) was added, followed by stirring. After completion of the reaction, methylene chloride and a 1N aqueous potassium hydroxide solution were added to the reaction mixture, followed by extraction. An organic layer was dried with sodium sulfate, filtered, and concentrated under reduced pressure. The residue obtained after concentration was dissolved by adding methylene chloride (2.5 mL), and then hexane (30 mL) was added to the resultant to precipitate a solid. The solid was filtered to obtain a target compound (383 mg, 75%) in the form of an orange solid.

R_{f} = 0.55 (EtOAc/Hexane, 1:1).

¹H NMR (400 MHz, CDCl₃) *δ* 0.84 -1.11 (28H, m, TIPDS), 3.28 (1H, dd, *J* = 4.5, 9.2 Hz), 3.67 (1H, dd, *J* = 2.0, 9.2 Hz), 4.03 (1H, d, *J* = 12.9 Hz), 4. 13 (1H, dd, *J* = 3.0, 12.9 Hz), 4.54 (1H, d, *J* = 4.4 Hz), 5.54 (1H, br s), 5.97 (1H, s), 6.25 (1H, br s), 9.13 (1H, s).

### Step 2: Preparation of 4-amino-1-((6aR,8R,9aS)-2,2,4,4-tetraisopropyltetrahydro-6H-thieno[3,2-f][1,3,5,2,4]trioxadisilocin-8-yl)-1,3,5-triazine-2(1H)-one

The compound (200 mg, 0.326 mmol) obtained in Step 1 and azobisisobutyronitrile (AIBN) (20 mg, 10 wt % starting material) were added to toluene (2 mL) and stirred to be uniformly mixed. Tributyltin hydride (Bu₃SnH) (0.26 mL, 0.98 mmol) was dissolved in toluene (6 mL) at 45°C under a nitrogen atmosphere, added dropwise for 20 minutes, heated to 60°C, and stirred for 1 hour and 30 minutes. After completion of the reaction, the reaction mixture was cooled to room temperature, a saturated aqueous ammonium chloride solution (10 mL) was added thereto, and an aqueous layer was extracted using ethyl acetate (10 mL x 3). An organic layer was extracted with a saturated aqueous sodium chloride solution (10 mL), dried with sodium sulfate, filtered, and concentrated under reduced pressure. Hexane was added to the concentrated residue to precipitate a solid. The solid was filtered to obtain a target compound (34 mg, 50%) in the form of a white solid.

R_{f} = 0.1 (DCM/MeOH, 10:1).

¹H NMR (400 MHz, CDCl₃) *δ* 0.87-1.13 (28H, m, TIPDS), 2.34 (1H, dd, *J* = 5.4, 13.5 Hz), 2.52 (1H, m), 3.36 (1H, m), 3.98 (1H, dd, *J* = 1.2, 12.7 Hz), 4.14 (1H, dd, *J* = 3.1, 12.8 Hz), 4.33 (1H, m), 5.45 (1H, br s), 5.90 (1H, br s), 5.97 (1H, dd, *J* = 6.7 Hz), 9.02 (1H, s).

### Step 3: Preparation of 4'-thio-5-aza-2'-deoxycytidine

The compound (101.5 g, 0.198 mol) obtained in Step 2 and ammonium fluoride (38.7 g, 1.045 mol) were added to methanol (1.47 L), heated to 60 to 65°C, and stirred for 2 hours. After completion of the reaction, the reaction mixture was cooled to 10 to 15°C and stirred for 30 minutes. The resulting solid was filtered and washed with methanol (60 mL x 2) to obtain a target compound (24.1 g, 60%) in the form of a white solid.

¹H NMR (400 MHz, DMSO-d₆) *δ* 2.18-2.31 (2H, m), 3.28 (1H, td, *J* = 3.5, 5.9 Hz), 3.54 (1H, dt, *J* = 5.4, 11.2 Hz), 3.63 (1H, dt, *J* = 5.9, 11.7 Hz), 4.33 (1H, q, *J* = 4.1 Hz), 5.15 (1H, t, *J* = 5.4 Hz), 5.26 (1H, d, *J* = 4.0 Hz), 6.08 (1H, t, *J* = 7.1 Hz), 7.55 (2H, d, *J* = 3.3 Hz), 8.70 (1H, s).

While the present invention has been described in detail with reference to specific features, it will be apparent to those skilled in the art that this description is illustrative only of the preferred embodiment and is not intended to limit the scope of the present invention. Therefore, the actual scope of the present invention is defined by the appended claims and their equivalents.

## Claims

1. A method for preparing 4'-thio-5-aza-2'-deoxycytidine, the method comprising:
(S-1) preparing a compound represented by the following Chemical Formula 2 from a compound represented by the following Chemical Formula 1;
(S-2) preparing a compound represented by the following Chemical Formula 3 from the compound represented by Chemical Formula 2; and
(S-3) preparing a compound represented by the following Chemical Formula I from the compound represented by Chemical Formula 3: 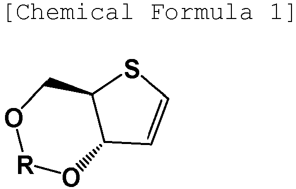 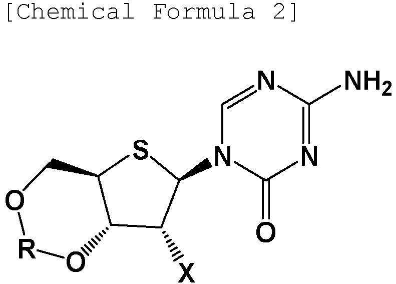 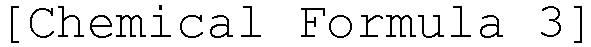 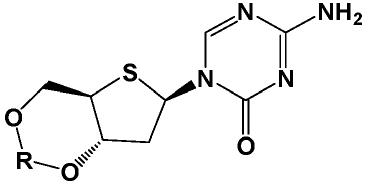 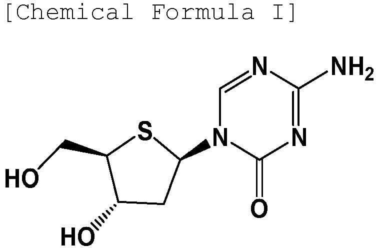
wherein R is a protecting group of diol including one or more Si atom, and
X is halo.

2. The method of claim 1, wherein the compound represented by Chemical Formula 1 is a compound represented by the following Chemical Formula 1a or a compound represented by the following Chemical Formula 1b: 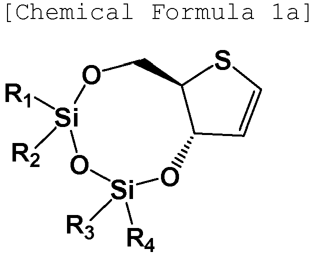 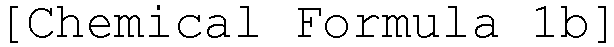 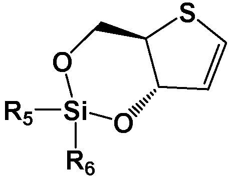 wherein R₁ to R₆ are each independently -C₁-C₆ alkyl, 3-7 membered cycloalkyl, or aryl.

3. The method of claim 1, wherein X is -bromo or -iodo.

4. The method of claim 1, wherein the step (S-1) is performed by reacting the compound represented by Chemical Formula 1 with 5-azacytosine and adding N-iodosuccinimide or N-bromosuccinimide thereto.

5. The method of claim 4, wherein the reaction between the compound represented by Chemical Formula 1 and 5-azacytosine is performed in the presence of hexamethyldisilane (HMDS).

6. The method of claim 4, wherein the reaction between the compound represented by Chemical Formula 1 and 5-azacytosine is performed in the presence of ammonium sulfate.

7. The method of claim 4, wherein N-iodosuccinimide or N-bromosuccinimide is added in the presence of trimethylsilyl trifluoromethanesulfonate (TMSOTf), tert-butyldimethylsilyl trifluoromethanesulfonate (TBDMSOTf), trifluoromethanesulfonic acid (TfOH), or boron trifluoride diethyl etherate (BF₃·OEt₂).

8. The method of claim 1, wherein the compound represented by Chemical Formula 1 is prepared from a compound represented by the following Chemical Formula 1c: 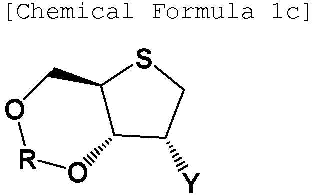 wherein Y is methanesulfonate (-OMs), trifluoromethanesulfonate (-OTf), or toluenesulfonate (-OTs).

9. The method of claim 8, wherein the compound represented by Chemical Formula 1c is prepared from a compound represented by the following Chemical Formula 1d:

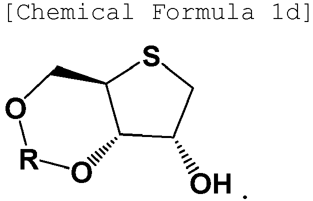

10. The method of claim 9, wherein the compound represented by Chemical Formula 1d is prepared from a compound represented by the following Chemical Formula 1e:

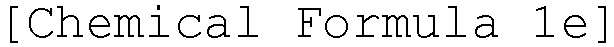

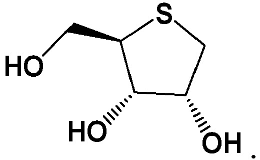

11. The method of claim 1, wherein the step (S-2) comprises reacting the compound represented by Chemical Formula 2 with a radical hydrogen donor in the presence of a radical initiator.

12. The method of claim 11, wherein the radical hydrogen donor is tributyltin hydride (Bu₃SnH), triphenyltin hydride (Ph₃SnH), or triethylborane (Et₃B).

13. The method of claim 11, wherein the radical initiator is 2,2'-azobisisobutyronitrile (AIBN), 1,1'-azobis(cyclohexanecarbonitrile)isobutyronitrile (ABCN), 2,2'-azobis-2,4-dimethylvaleronitrile (ADMVN), or 2,2'-azobis(4-methoxy-2,4-dimethylvaleronitrile (V-70).

14. The method of claim 1, wherein the step (S-3) is performed through a deprotection reaction.

15. The method of claim 14, wherein the step (S-3) is performed by adding ammonium fluoride (NH₄F), tetra-N-butylammonium fluoride (TBAF), hydrogen fluoride-pyridine (1:1), or triethylamine trihydrofluoride (TREAT-HF).

16. The method of claim 1, wherein the step (S-3) further comprises a crystallization process.
